# EUROPÄISCHE PATENTSCHRIFT

(11) **EP 1 621 882 B1**
(45) Veröffentlichungstag und Bekanntmachung des Hinweises auf die Patenterteilung: **21.10.2009**
(21) Anmeldenummer: 05011919.7
(22) Anmeldetag: 02.06.2005
(51) Int. Cl.: G01N 33/00, G01N 27/12

(54) **Verfahren zur Erfassung brennbarer Gase, insbesondere zur Erfassung von Wasserstoff**
Method for detecting combustible gases, in particular hydrogen
Procédé de détection des gaz combustibles et d'hydrogène en particulier

(30) Priorität: 03.06.2004 DE 102004027267; 06.12.2004 DE 102004058836
(43) Veröffentlichungstag der Anmeldung: 01.02.2006
(73) Patentinhaber: UST Umweltsensortechnik GmbH, 98716 Geschwenda (DE)
(72) Erfinder: Kiesewetter, Olaf, 98716 Geschwende (DE); Ewert, Anatolij, 98693 Ilmenau (DE); Melchert, Volkmar, 98693 Martinroda (DE); Kittelmann, Sven, 99326 Stadtilm (DE)
(74) Vertreter: Liedtke, Klaus

(56) Entgegenhaltungen:
- WO-A-01/18500
- DE-A1- 19 634 690
- FR-A1- 2 577 320
- JP-A- 8 327 576
- JP-A- 9 049 814
- US-B1- 6 513 364

## Beschreibung

Die Erfindung betrifft ein Verfahren zur Erfassung brennbarer Gase, insbesondere zur Erfassung von Wasserstoff, durch Auswertung von Signalen eines gasempfindlichen Halbleitergassensors.

Das Verfahren dient zur Ermittlung der Konzentration von Wasserstoff und anderen brennbaren Gasen, um bei Überschreiten eines Sollwertes ein auswertbares Signal abzugeben. Dabei ist es erforderlich, dass das Verfahren in einem Temperaturbereich von -50°C bis +150°C eine sichere Detektion ermöglicht.

Durch die zu erwartende zunehmende Nutzung von Wasserstoff als Energieträger gewinnt die quantitative Erfassung der Wasserstoffkonzentration sowie die Detektion von explosiven Wasserstoff-Luftgemischen in einem sehr großen Temperaturbereich mit einer Langzeitstabilität von mehr als 10 Jahren wachsende Bedeutung.

Es ist bekannt, zur Detektion von Wasserstoff verschiedene chemisch-physikälische Effekte zu nutzen.

Ein häufig angewandtes Verfahren verwendet elektrochemische Messzellen, die sehr selektiv und genau messen können, aber aufgrund hoher Temperaturempfindlichkeit und Austrocknungseffekten für viele Anwendungsgebiete, z. B. im Freien oder in Kraftfahrzeugen, nur mit großem Aufwand einsetzbar sind.

Ferner sind Sensoren bekannt, die den Temperaturanstieg bei der katalytischen Umsetzung von Wasserstoff und Luft an einem Einzelelement oder an einem Kombielement zur Signalerzeugung nutzen. Hierzu werden Vergleichselemente mit und ohne katalytische Zusätze eingesetzt. Nachteilig ist dabei, dass Vergifungserscheinungen und damit Fehlfunktionen auftreten können.

Materialien wie bestimmte Ni-Legierungen oder auch Metallhydride ändern ihren spezifischen elektrischen Widerstand, wenn Wasserstoff von der Schicht absorbiert wird. Nachteilig ist ferner, dass sich durch Änderung der Oberflächenzusammensetzung auch die Kennlinien dieser Effekte stark ändern und das Signal durch Temperaturänderungen sehr stark beeinflusst wird.

Es ist auch bekannt, Sensoren zu verwenden, die durch Absorption von Wasserstoff in das Metallgefüge ihr Volumen ändern und mit Hilfe einer Bimetallanordnung ein Signal erzeugen.

So genannte Wärmeleitsensoren, die hochgenaue Signale durch Auswertung der Wärmeleitfähigkeit von Gasen erzeugen, sind zwar in einem weiten Temperaturbereich einsetzbar, haben aber den Nachteil, dass Ihr Signal in Gasgemischen mit mehreren Gasen undefiniert sein kann, da die Wärmeleitung verschiedener Gase gegenüber Luft nicht immer eine eindeutige Aussage über das vorhandene Gasgemisch erlaubt.

Nach DE 199 59 699 C1 ist eine Vorrichtung zur Überwachung der Wasserstoffkonzentration in einem Medium bekannt, bei dem Wärmeleitfähigkeitssensoren zur Wasserstoffdetektion verwendet werden. Die gemessene Wärmeleitfähigkeit wird durch eine Signalaufbereitung in eine elektrische Spannung umgewandelt und mit einem zulässigen Wert verglichen. Zur Verringerung von Messfehlem und Fehlsignalen wird vorgeschlagen, bei Erreichen des zulässigen Grenzwertes mehrfach zu prüfen, ob der Zustand über einen längeren Zeitraum anliegt.

Ein weiteres Gassensorelement, welches sich insbesondere für Untersuchungen im Spurenbereich als sehr vorteilhaft erwiesen hat, arbeitet auf der Basis von halbleitenden Metalloxiden. Nachteilig ist hierbei, dass die Selektivität und Empfindlichkeit gegenüber Vergiftungen sehr hoch ist. Nachteilig ist ferner, dass die Genauigkeit im oberen Konzentrationsbereich recht niedrig ist und die Kennliniensteilheit des Sensors bei ca. 1-2 Vol% sehr flach verläuft.

Um einige Nachteile der genannten Funktionsprinzipien zu umgehen, wurden Kombinationsmethoden vorgeschlagen. So wurden Halbleitergassensoren in Brückenschaltung bezüglich des Pellistoreffektes ausgewertet. Auch wurden katalytische Effekte mit Metallhydrid-Schichten und Wärmeleitsensoren mit verschiedenen Brückenschaltungen kombiniert.

Nach DE 198 19 575 C1 ist ein Wasserstoffsensor mit einer temperaturabhängig wasserstoffempfindlichen Halbleiterschicht aus Strontiumtitanat SrTiO₃ und einer wasserstoff durchlässigen Deckschicht bekannt, bei dem zur Kompensation der Temperaturabhängigkeit eine weitere Strontiumtitanatschicht angeordnet ist, die von einer selektiv filternden Deckschicht abgedeckt ist und einen anderen Verlauf der Leitfähigkeitskennlinie als die erste Halbleiterschicht aufweist.

In JP 08-3 27 576 A ist eine Anordnung beschrieben, bei der ein Wärmeleitsensor, der bei hoher Gaskonzentration reagiert, mit einem Halbleiter-Gassensor kombiniert wurde, der bei geringer Gaskonzentration anspricht. Nachteilig ist dabei, dass diese Anordnung nicht zur Detektion von Wasserstoff in tertiären Gemischen geeignet ist und bei der Detektion von Gasgemischen die Gefahr von Signalauslöschungen besteht.

Weitere Möglichkeiten sind in JP 09 049814 A, FR 2 577 320 A1 und JP 08 327576 A beschrieben.

Allen bekannten Einzelanordnungen ist gemeinsam, dass sie entweder nur eine geringe Selektivität für Wasserstoff oder nur eine geringe Empfindlichkeit aufweisen und deshalb nur ungenaue Messungen ermöglichen. Zur Erhöhung der Messgenauigkeit werden deshalb mehrere kostenaufwendige Sensorelemente kombiniert. Für industrielle Anwendungen sind kostenaufwendige Anordnungen in der Regel nicht nachteilig, da der Preis des Elements für den Preis des Systems nicht relevant ist. Für Anwendungsfälle, die Sensoren in großen Stückzahlen erfordern, beispielsweise für den Einsatz im Automobilbereich sowie in Gebäuden sind jedoch kostengünstige Ausführungen zwingend erforderlich.

Der Erfindung liegt die Aufgabe zugrunde, ein Verfahren der eingangs genannten Art anzugeben, mit dem kostengünstig eine hohe Messgenauigkeit ermöglicht wird.

Erfindungsgemäß wird die Aufgabe mit einem Verfahren, welches die in Anspruch 1 angegebenen Merkmale aufweist, gelöst.

Vorteilhafte Ausgestaltungen sind in den zugehörigen Unteransprüchen angegeben.

Mit dem erfindungsgemäßen Verfahren gelingt es, die geringe Selektivität eines Wärmeleitfähigkeitssensors zu verbessern, indem dieser mit einem speziellen Metalloxid-Halbleitergassensor kombiniert wird. Dieses Verfahren kann schon bei sehr niedrigen Temperaturen betrieben werden und zeichnet sich trotzdem durch eine hohe selektive Empfindlichkeit aus. Das Verfahren ermittelt die Gaskonzentration sehr genau, weist aber nur eine geringe selektive Empfindlichkeit für die hier in Betracht kommenden Gase auf.

Die beiden Sensoren können einzeln oder in einem gemeinsamen Gehäuse angeordnet sein. Der Wärmeleitfähigkeitssensor verfügt über zwei Temperatursensorelemente, die durch eine Wärmebrücke verbunden sind.

Zur Erfassung der Wasserstoffkonzentration ist der Halbleitergassensor mit einem Temperatursensor verbunden und wird auf eine bestimmte Temperatur über Umgebungstemperatur aufgeheizt, wobei der Gassensor das Selektivitäts- und Nullpunktsignal für die Wärmleitfähigkeitsbestimmung darstellt.

Damit gelingt es, für einen Wärmeleitdetektor das Start- und Nullpunktsignal im Bereich einiger weniger ppm zu legen. Damit kann auch bei komplexen Gasgemischen im Vol%-Bereich ein hochgenaues quantitatives Signal gewonnen werden.

Das Sensorgehäuse ist dabei so gestaltet, dass der Wärmewiderstand der Wärmebrücken und der Wärmewiderstand des den Sensor umgebenden Luftvolumens in einem definierten Verhältnis stehen.

Die Erfindung wird im Folgenden an einem Ausführungsbeispiel näher erläutert. Die zugehörigen Zeichnungen zeigen Anordnungen zur Durchführung des Verfahrens. Dabei sind dargestellt in
- Figur 1: eine Anordnung in gekapselter Ausführung mit einem Chip,
- Figur 2: eine Anordnung in gekapselter Ausführung mit zwei Chips,
- Figur 3: eine Anordnung in gekapselter Ausführung mit zwei Chips und einer thermischen Isolierung,
- Figur 4: eine Anordnung, bei der die beiden Sensoren jeweils in einem eigenen SMD-Gehäuse angeordnet sind, wobei sich der Temperatursensor mittig unterhalb von Wärmeleitfähigkeitssensor und Gassensor befindet,
- Figur 5: eine Anordnung gemäß Figur 4 mit einer zusätzlichen Membran am Wärmeleitfähigkeitssensor,
- Figur 6: eine Anordnung, bei der die beiden Sensoren jeweils in einem eigenen SMD-Gehäuse angeordnet sind, wobei sich der Temperatursensor unterhalb des Wärmeleitfähigkeitssensors befindet,
- Figur 7: eine Anordnung, bei der die beiden Sensoren jeweils in einem eigenen SMD-Gehäuse angeordnet sind, wobei sich der Temperatursensor unterhalb des Wärmeleitfähigkeitssensors befindet und der Gassensor einen zusätzlichen Boden aufweist,
- Figur 8: eine Anordnung, bei der die beiden Sensoren jeweils in einem eigenen SMD-Gehäuse getrennt vergossen und einzeln angeordnet sind,
- Figur 9: eine Ausführung, bei der die Sensoren in flacher Anordnung auf einem Substrat angebracht sind, wobei der Halbleitersensor, Temperatursensor und Heizer in einer Schicht integriert sind,
- und Figur 10: eine Ausführung, bei der die Sensoren in flacher Anordnung auf einem Substrat angebracht sind und der Halbleitersensor mit einem Temperatursensor in einer Schicht integriert ist.

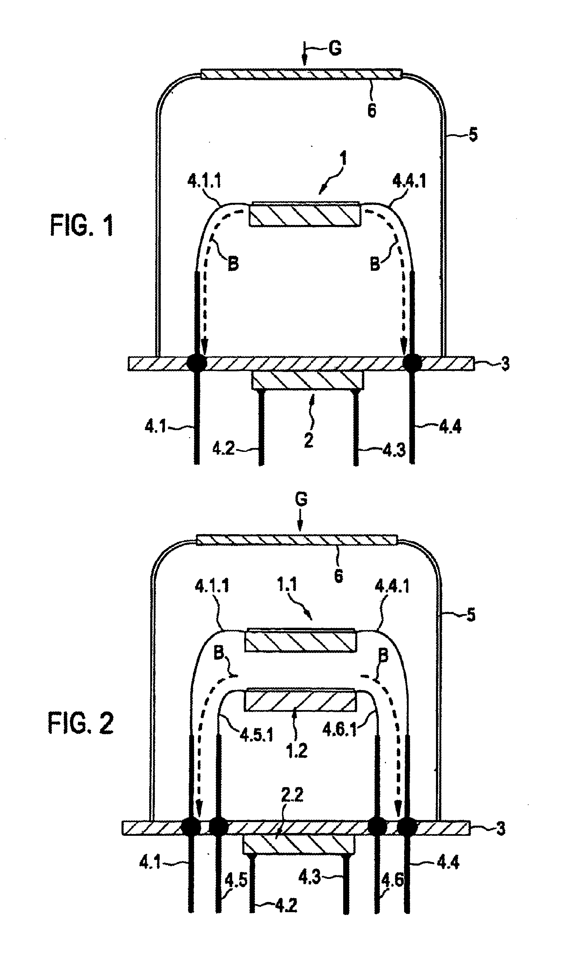

Bei der in Figur 1 dargestellten Anordnung sind ein Halbleitergassensor (GS) und ein Wärmeleitfähigkeitssensor (WS) in einem gemeinsamen Gehäuse (5) angeordnet. Dabei ist auf einem Träger 3 das Gehäuse 5 angebracht, in dem sich oberhalb des Trägers 3 ein Chip 1 befindet, welches eine spezielle wasserstoffempfindliche Metalloxidschicht, eine Heizeinrichtung und einen inneren Temperatursensor enthält. Als wasserstoffempfindliche Metalloxidschicht hat sich ein speziell dotiertes Zinndioxid besonders bewährt, es sind aber auch andere Metalloxide einsetzbar. Es sind auch Ausführungen möglich, bei denen ein Heizwiderstand angeordnet ist, der gleichzeitig als Temperaturmesswiderstand dient. Ferner ist es möglich, dass der Chip 1 neben der sensitiven Schicht einen Heizwiderstand und zwei Temperaturmesswiderstände enthält. Die Verwendung von zwei Temperaturmesswiderständen, die vorzugsweise in einer Reihenschaltung angeordnet sind, ermöglicht das Erkennen von Verschmutzungen auf dem Chip. Der Chip 1 ist an Drähten freitragend im zu untersuchendem Medium aufgehängt. Im dargestellten Beispiel ist der Chip 1 über als Wärmebrücke B wirkende elektrische Verbindungsleitungen 4.1.1 und 4.1.4 mit den durch den Träger 3 führenden elektrischen Anschlüssen 4.1 und 4.4 verbunden. Ferner führen zwei weitere - hier nicht dargestellte Verbindungsleitungen - zu zwei weiteren Anschlüssen, die ebenfalls in der Figur nicht gezeigt sind. Das Gehäuse 5 ist an der dem Träger 3 gegenüber liegenden Oberseite mit einer Membran 6 versehen, durch die das zu überwachende Gas G in das Gehäuse 5 gelangt. Der Chip 1 befindet sich etwa in der Mitte zwischen Träger 3 und Oberseite des Gehäuses 5 und definiert mit seiner Lage ein bestimmtes Verhältnis des Wärmeleitvermögens, welches durch das im Inneren des Gehäuses 5 sich befindende Gas bestimmt wird, und des Wärmeleitvermögens, welches durch die Wärmebrücke B realisiert wird. Unterhalb des Trägers 3 befindet sich der äußere Temperatursensor 2, mit dem die Umgebungstemperatur bestimmt werden kann. Der Temperatursensor 2 ist über die elektrischen Anschlüsse 4.2 und 4.3 nach außen kontaktierbar. Die Anordnung kann über insgesamt sechs Anschlüsse, von denen in der Figur die Anschlüsse 4.1 ... 4.4 ersichtlich sind, mit einer hier nicht dargestellten Auswerteeinheit verbunden werden. Wenn der Chip 1 durch seine Heizvorrichtung erhitzt wird, dann wird die Wärme einerseits über das zu untersuchende Gas und andererseits durch die Wärmebrücke B abgeleitet.
Die Wärmeleitfähigkeit der Wärmebrücke B wird durch Länge und Durchmesser der Verbindungsleitungen und der Anschlüsse sowie deren Materialeigenschaften bestimmt. Durch die Dimensionierung der Wärmebrücke B kann das Verhältnis des Wärmestroms, der durch das Gasvolumen fließt, zum Wärmestrom, der über die Wärmebrücke B fließt, festgelegt werden.
Aus dem Temperaturunterschied, der zwischen dem Heizwiderstand, welchen der Chip 1 enthält, und dem Temperatursensor 2 messbar ist, wird ein Wärmeleitfähigkeitssignal gewonnen.

Bei der in **Figur 2** gezeigten Ausführung sind zwei Chips 1.1 und 1.2 übereinander angeordnet, wobei im oberen Chip 1-1 die Metalloxidschicht und der Heizer angeordnet sind und im unteren Chip 1.2 ein Temperatursensor zur Ermittlung der Temperatur des sich im Gehäuse 5 befindenden Gases G angebracht ist. Der Chip 1.1 ist über die Verbindungsleitungen 4.1.1 und 4.4.1 thermisch und elektrisch mit den Anschlüssen 4.1 und 4.4 und zwei weiteren Anschlüssen verbunden, während der Chip 1.2 in gleicher Weise durch die Verbindungsleitungen 4.5.1 und 4.6.1 mit den Anschlüssen 4.5 und 4.6 verbunden ist. Die Verbindungsleitungen bilden in gleicher Weise, wie oben erläutert, eine Wärmebrücke B. Diese Anordnung gestattet es, den oberen Chip 1.1 einer höheren Temperatur auszusetzen, ohne dass im Gehäuse eine unzulässige Gastemperatur entsteht, so dass ein größeres Temperaturgefälle und damit eine höhere Genauigkeit erreicht werden kann.

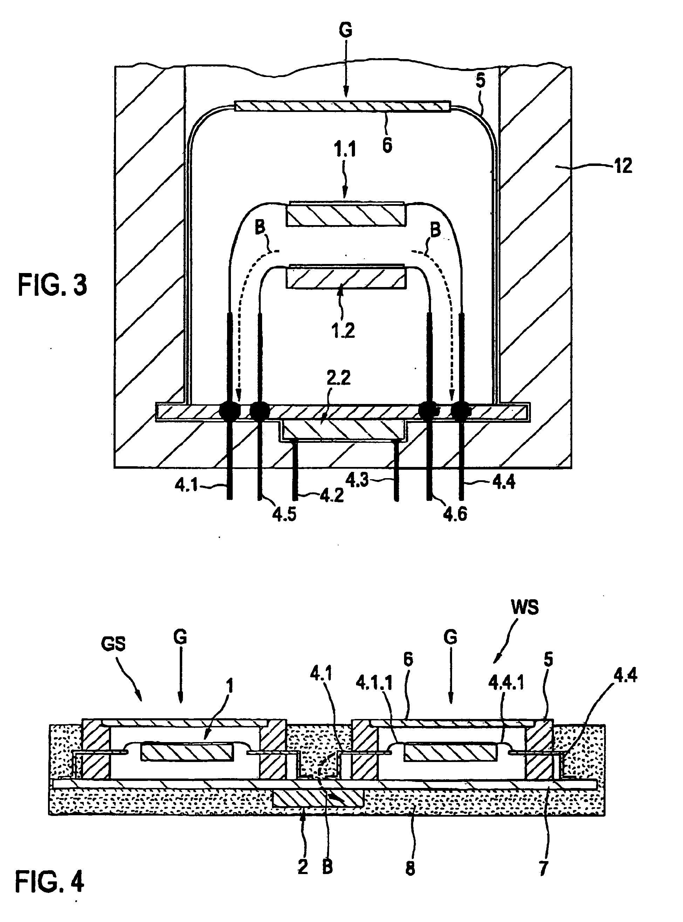

Eine vorteilhafte Ausgestaltung entsteht dadurch, dass das Gehäuse 5 zur Stabilisierung der Temperaturverhältnisse mit einer thermischen Isolierung umgeben ist, wie dies in **Figur 3** dargestellt ist.

**Figur 4** erläutert eine Anordnung, bei der der Halbleitergassensor GS und der Wärmeleitfähigkeitssensor WS jeweils in einem eigenen SMD-Gehäuse 5 nebeneinander auf dem Substrat 7 angeordnet sind. Das Substrat 7 besteht vorzugsweise aus Keramik, Metall oder handelsüblichen Leiterplattenmaterial. An der Unterseite des Substrates 7 ist der Temperatursensor 2 etwa mittig unterhalb von Wärmeleitfähigkeitssensor WS und Halbleitergassensor GS angebracht. Die Gesamtanordnung ist mit einem Verguss 8 geschützt, wobei die jeweils mit einer Membran 6 abgeschlossenen Oberseiten der Sensoren GS und WS aus den Verguss 8 herausragen. Die Sensoren GS und WS sind mit den Anschlüssen 4 verbunden, wobei durch die Verbindung von Chip 1 des Wärmeleitfähigkeitssensor WS durch seine Verbindungsleitungen 4.1.1 und 4.4.1 und elektrischen Anschlüsse 4.1 und 4.4 mit dem Substrat 7 eine Wärmebrücke B zum Temperatursensor 2 gebildet wird. Wärmeleitfähigkeitssensor WS und Halbleitergassensor GS sind an ihrer Oberseite durch eine Membran 6 abgedeckt.

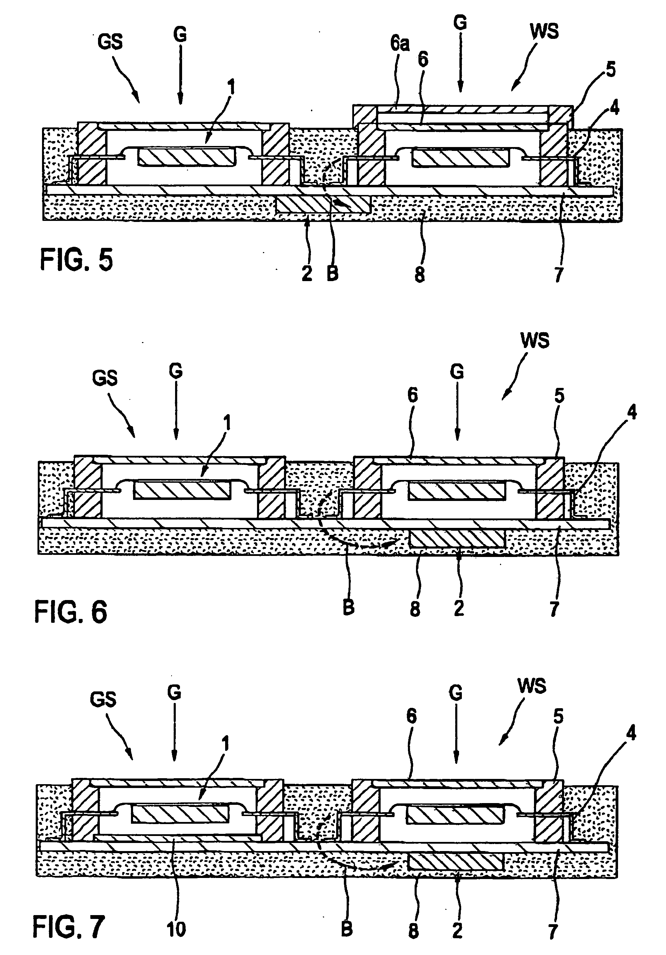

Eine in **Figur 5** dargestellte vorteilhafte Ausführung ist oberhalb der Membran 6 mit einer weiteren Membran 6.1 versehen, mit der ein zusätzlicher Diffusionsbereich geschaffen und die Windempfindlichkeit des Wärmeleitfähigkeitssensors WS verringert wird.

Bei der in **Figur 6** gezeigten Anordnung befindet sich der Temperatursensor 2 unterhalb des Wärmeleitfähigkeitssensors WS. Dadurch können die Parameter der Wärmebrücke B optimal auf die Eigenschaften des Wärmeteitfähigkeitssensors WS abgestimmt und Quereinflüsse verringert werden.

In **Figur 7** ist eine Ausführungsform dargestellt, bei der die beiden Sensoren GS und WS sowie der Temperatursensor 2 in der bei Figur 6 beschriebenen Weise angeordnet sind. Der Halbleitergassensor GS ist hier mit einen zusätzlichen Boden 10 versehen. Dadurch kann der Einfluss störender Wärmeeinträge, die vom Gassensor verursacht werden, verringert werden.

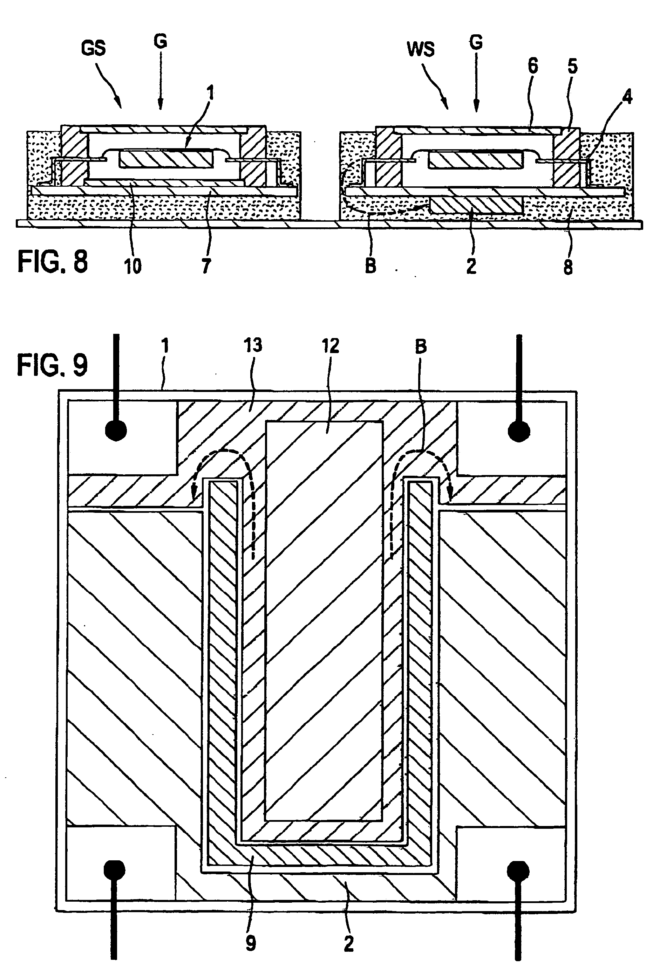

Eine weitere Möglichkeit der Sensoranordnung ist aus **Figur 8** ersichtlich. Bei dieser Anordnung sind die beiden Sensoren GS und WS jeweils in einem eigenen SMD-Gehäuse getrennt in je einem Verguss 8 fixiert. Der Temperatursensor 2 befindet sich unterhalb des Wärmeleitfähigkeitssensors WS und der Halbleitergassensor GS weist einen zusätzlichen Boden 10 auf. Die beiden Einzelsensoren GS und WS können getrennt untergebracht werden, müssen aber dem gleichen zu untersuchenden Gas G ausgesetzt sein. Im dargestellten Beispiel sind sie nebeneinander auf einem Befestigungsteil 11 angeordnet.

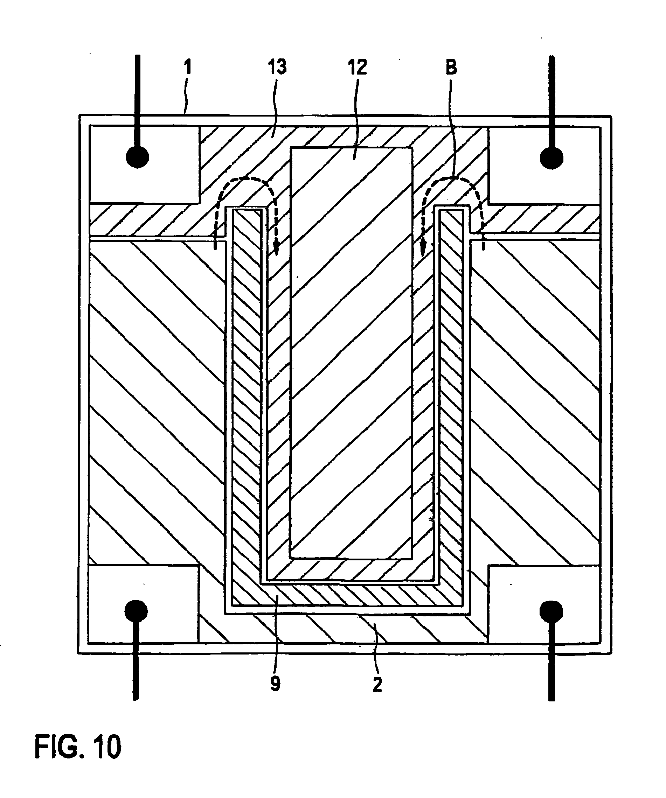

In den **Figuren 9** **und** **10** sind Ausführungsformen dargestellt, bei denen die Sensoren GS und W S in einer flachen Gesamtanordnung auf einem Chip 1 angebracht sind.
Dabei sind bei der in Figur 9 gezeigten Ausführung die Metalloxidschicht 12 des Halbleitergassensors GS, ein Widerstandselement 13, das den Temperatursensor und eine Heizeinrichtung enthält, in einer ersten Schicht integriert. Der Temperatursensor 2 befindet sich in einer zweiten Schicht, die in der gleichen Ebene auf dem Chip 1 angeordnet ist und von der ersten Schicht durch einen Trenngraben 9 getrennt ist, welcher den gasempfindlichen wärmewiderstand des Wärmeleitfähigkeitssensors darstellt.
Demgegenüber sind bei der Ausführung gemäß Figur 10 Halbleitergassensor GS und Temperatursensor in einer ersten Schicht integriert, die von einer zweiten Schicht, welche Temperatursensor und Heizeinrichtung enthält, durch den Trenngraben 9 getrennt ist.
Bei den in den Figuren 9 und 10 dargestellten Anordnungen werden die Wärmeleitwerte der Wärmebrücken B durch die Abstände zwischen den beiden Schichten bestimmt.

### BEZUGSZEICHENLISTE

- G: Gas
- GS: Halbleitergassensor
- WS: Wärmeleitfähigkeitssensor
- B: Wärmebrücke

- 1: Chip
1.1 oberer Chip
1.2 unterer Chip
- 2: äußerer Temperatursensor
- 3: Träger
- 4: elektrische Anschlüsse
4.1.1, 4..4.1, 4.5.1, 4.6.1 Verbindungsleitungen
- 5: Gehäuse
- 6: Membran
- 7: Substrat
- 8: Verguss
- 9: Trenngraben, gasempfindlicher wärmewiderstand
- 10: Boden
- 11: Befestigungsteil
- 12: Metalloxidschicht des Halbleitergassensors
- 13: Widerstandselement für Temperatursensor und Heizung

## Patentansprüche

1. Verfahren zur Erfassung von Wasserstoff durch Auswertung von Signalen eines gasempfindlichen Halbleitersensors (GS), **dadurch gekennzeichnet, dass** der Halbleitergassensor (GS) auf eine bestimmte Temperatur über Umgebungstemperatur aufgeheizt wird, wobei die Temperatur mit einem temperaturempfindlichen Heizer erfasst wird, und gleichzeitig Signale des Halbleitergassensor (GS) und Signale eines Wärmeleitfähigkeitssensor (WS) erfasst werden und die Signale des Halbleitergassensors (GS) und des Wärmeleitfähigkeitssensors (WS) in einer gemeinsamen Auswerteeinheit verarbeitet werden, wobei der Halbleitergassensor (GS) ein Selektivitäts- und Nullpunktsignal für den Wärmeleitfähigkeitssensor (WS) abgibt, indem der Wärmeleitfähigkeitssensor (WS) in einen Arbeitsbereich hoher Empfindlichkeit gebracht wird, nachdem der Halbleitergassensor (GS) ein Signal für die Erkennung von Wasserstoff abgegeben hat, und aus beiden Signalen die Konzentration von Wasserstoff ermittelt wird.

2. Verfahren nach Anspruch 1, **dadurch gekennzeichnet, dass** bei Bestätigung der Detektion des zu detektierenden Gases durch den Wärmeleitfähigkeitssensor (WS) ein Alarmsignal ausgelöst wird.

3. Verfahren nach Anspruch 1 oder 2, **dadurch gekennzeichnet, dass** die Signale des Halbleitergassensors (GS) und des Wärmeleitfähigkeitssensors (WS) mit Hilfe von Wichtungsfunktionen verknüpft werden.

4. Verfahren nach einem der vorhergehenden Ansprüche, **dadurch gekennzeichnet, dass** mindestens einer der Sensoren (GS, WS) in zwei unterschiedlichen Temperaturbereichen betrieben wird, wobei die Anordnung entweder mit niedriger Temperatur in low-power-Modus bei geringerer Genauigkeit oder im Normalmodus bei höherer Temperatur mit höherer Genauigkeit betrieben wird.

5. Verfahren nach einem der vorhergehenden Ansprüche, **dadurch gekennzeichnet, dass** zur Vermeidung katalytischer Effekte die Temperatur am Wärmeleitfähigkeitssensors (WS) um einen definierten Betrag unterhalb der Temperatur gehalten wird, bei der katalytische Effekte eintreten.

6. Verfahren nach einem der vorhergehenden Ansprüche, **dadurch gekennzeichnet, dass** die Umgebungstemperatur erfasst und der Auswerteeinheit zugeführt wird.

## Claims

1. Method for detecting hydrogen by analyzing signals of a gas-sensitive semiconductor sensor (GS), **characterized in that** the semiconductor gas sensor (GS) is heated to a specified temperature above the ambient temperature, wherein the temperature is acquired by means of a temperature sensitive heater and wherein signals of the semiconductor gas sensor (GS) and signals of a heat conductivity sensor (WS) are acquired simultaneously and wherein the signals of the semiconductor gas sensor (GS9 and the heat conductivity sensor (WS) are processed in a joint analyzing unit, wherein the semiconductor gas sensor (GS) emits a selectivity and zero point signal for the heat conductivity sensor (WS) by bringing the heat conductivity sensor (WS) into an operating range with high sensitivity after the semiconductor gas sensor (GS) has emitted a signal for detecting hydrogen and wherein the concentration of hydrogen is determined from both signals.

2. Method according to claim 1, **characterized in that** an alarm signal is released upon a confirmation of the detection of the gas to be detected by the heat conductivity sensor.

3. Method according to claim 1 or 2, **characterized in that** the signals of the semiconductor gas sensor (GS) and of the heat conductivity sensor (WS) are combined by means of weighting functions.

4. Method according to one of the preceding claims, **characterized in that** at least one of the sensors (GS, WS) is operated in two different temperature ranges, wherein the arrangement is either operated at low temperature in low power mode and with lower accuracy or in normal mode at higher temperature with higher accuracy.

5. Method according to one of the preceding claims, **characterized in that**, in order to avoid catalytic effects, the temperature at the heat conductivity sensor (WS) is kept by a defined value below the temperature, at which catalytic effects occur.

6. Method according to one of the preceding claims, **characterized in that** the ambient temperature is acquired and fed to the analyzing unit.

## Revendications

1. Procédé de saisie d'hydrogène par analyse de signaux d'un capteur à semi-conducteur sensible aux gaz (GS), **caractérisé en ce que** le capteur de gaz à semi-conducteur (GS) est chauffé à une température définie au-dessus de la température ambiante, ladite température étant déterminée par un filament sensible à la chaleur, des signaux du capteur de gaz à semi-conducteur (GS) et des signaux d'un capteur de conductivité thermique (WS) étant saisis simultanément, et les signaux du capteur de gaz à semi-conducteur (GS) et du capteur de conductivité thermique (WS) étant traités dans une unité d'analyse commune, le capteur de gaz à semi-conducteur (GS) délivrant un signal de sélectivité et de point zéro pour le capteur de conductivité thermique (WS) par amenée du capteur de conductivité thermique (WS) dans une plage de travail à haute sensibilité, après délivrance d'un signal pour la détection d'hydrogène par le capteur de gaz à semi-conducteur (GS), et **en ce que** la concentration d'hydrogène est déterminée à partir des deux signaux.

2. Procédé selon la revendication 1, **caractérisé en ce qu'**un signal d'alarme est déclenché en cas de confirmation de détection du gaz à détecter par le capteur de conductivité thermique (WS).

3. Procédé selon la revendication 1 ou la revendication 2, **caractérisé en ce que** les signaux du capteur de gaz à semi-conducteur (GS) et du capteur de conductivité thermique (WS) sont liés au moyen de fonctions de pondération.

4. Procédé selon l'une des revendications précédentes, **caractérisé en ce qu'**au moins un des capteurs (GS, WS) est mis en service dans deux plages de températures différentes, l'agencement étant mis en service soit à basse température en mode à faible puissance avec une précision réduite, soit en mode normal à température élevée avec une haute précision.

5. Procédé selon l'une des revendications précédentes, **caractérisé en ce que**, pour éviter des phénomènes catalytiques, la température est maintenue sur le capteur de conductivité thermique (WS) avec un écart défini en dessous de la température où apparaissent des phénomènes catalytiques.

6. Procédé selon l'une des revendications précédentes, **caractérisé en ce que** la température ambiante est déterminée et transmise à l'unité d'analyse.
